Europäisches Patentamt

European Patent Office

Office européen des brevets

(11) Publication number: **0 130 708**
**A1**

(12) # EUROPEAN PATENT APPLICATION

(21) Application number: **84303827.4**

(22) Date of filing: **06.06.84**

(51) Int. Cl.⁴: **G 01 N 33/96,** G 01 N 33/50, C 12 Q 1/32, C 12 Q 1/34, C 12 Q 1/48

(30) Priority: **06.06.83 US 501213**
**06.06.83 US 501216**
**06.06.83 US 501358**
**29.06.83 US 501222**

(43) Date of publication of application: **09.01.85**
**Bulletin 85/2**

(84) Designated Contracting States: **AT BE CH DE FR GB IT LI NL SE**

(71) Applicant: **ORTHO DIAGNOSTIC SYSTEMS INC., One Johnson & Johnson Plaza, New Brunswick New Jersey 08933 (US)**

(72) Inventor: **Vail, Martha, P.O. Box 595 Ventura, California 93002 (US)**
Inventor: **Megraw, Robert Ellis, 14442, Deerfield Avenue, Tustin CA 92680 (US)**
Inventor: **Hoskins, Michael Kevin, 15191 Champagne Circle, Irvine CA 92714 (US)**

(74) Representative: **Jones, Alan John et al, CARPMAELS & RANSFORD 43 Bloomsbury Square, London, WC1A 2RA (GB)**

(54) **Stabilized clinical control reagents.**

(57) This invention relates to stabilized clinical control reagents, such as multiparameter control reagents useful for calibrating clinical chemistry analysers and for manual testing methods, isoenzyme control reagents including, for instance, CK and LDH isoenzyme, coagulation control reagents, plasma renin control reagents, and reagents for use in steriod receptor analysis.

The present invention provides a stabilized clinical control reagent comprising an aqueous solution of biological analyte and sufficient plexiform stabilizing means selected from glucose, sucrose, reducing monosaccharide sugars and reducing disaccharide sugars to obtain substantially increased analyte stability over that obtained without such plexiform stabilizing means.

−1−

## STABILIZED CLINICAL CONTROL REAGENTS

This invention relates to stabilized clinical control reagents, such as multiparameter control reagents useful for calibrating clinical chemistry analysers and for manual testing methods, isoenzyme control reagents including, for instance, CK and LDH isoenzymes, coagulation control reagents, plasma renin control reagents, and reagents for use in steriod receptor analysis.

In particular, the invention relates to clinical control reagents and methods for producing such reagents which are suitable for lyophilisation or spray drying.

## Background of the Invention

Chemistry analyzers occupy a preeminent position in the clinical environment as they provide significant data regarding the diagnosis and treatment of patient illnesses. Accordingly, there has been a concerted effort by many investigators to develop automated and manual methods for the determination and qualification of constituents in body fluids such as acid phosphatase, alanine aminotransferase, albumin, aldolase, alkaline phosphatase, alpha-hydroxybutyrate dehydrogenase, amylase, aspartate aminotransferase, bicarbonate, bilirubin direct, bilirubin total, blood urea nitrogen, calcium total, carbon dioxide, chloride, total cholesterol, cholinesterase, cortisol, creatine kinase, creatinine, digoxin, gamma glutamyl-transferase, globulin, glucose, hdl-cholesterol, iron, lactate dehydrogenase-1, lactate dehydrogenase-p, lactic acid, lypase, lithium, magnesium, osmolality, phenylalanine, phosphorus, potassium, salicylate, sodium, T3, T3 uptake, T4, total iron binding capacity (TIBC), total lipids, total protein, triglycerides, UIBC, and uric acid, to name a few.

Both the manual and automated methods available for each
one of the above analytes and constituents have in common
the requirement for a control reagent whereby the procedures and other variable parameters may be checked to ensure accuracy of the testing method or instrument.

It is an object of the present invention to provide such a control reagent useful for automated and manual methods.

Although  multiparameter control reagents are known and include the Ortho™ Normal Control Serum-Assayed, available from Ortho Diagnostic Systems Inc., Raritan, New Jersey, all such reagents have suffered, albeit to different extents, from stability related deficiencies. Specifically, these reagents have heretofor been characterized by their limited shelf lives, both in their typically dry storage state and in their liquid testing format. For instance, $CO_2$ levels and enzyme constituents are particularly prone to decomposition and deactivation. Accordingly, the clinical researcher has heretofore been generally forced to prepare fresh chemistry control reagents for each testing batch or work shift as appropriate. The unfortunate consequence of this requirement is the increase in potential errors as well as the concomitant costs incurred.

It is another object of the instant invention to provide clinical chemistry control reagents having greatly increased stability whereby many of the above related stabilization problems are obviated.

Conventional attempts to prolong stability of chemistry control reagents and related reagents include reduction of the control reagent to a dry format which is then typically refrigerated at approximately 4°C. Often, the dry format is accomplished by lyophilization rather than spray drying as the former operates at far lower temperatures than the latter. It is known that heat disadvantageously aggravates degradation of analytes and proteins in

0130708

-3-

general and accordingly cold processes such as freeze concentration and freeze drying are generally preferred. Although stability in a lyophilized state is enhanced over that associated with the aqueous format, significant losses in constituent activity levels of known chemistry control reagents are still incurred.

It is a yet further object of the present invention to provide methods and reagents which provide the required stability levels for substantially all constituents and analytes in a clinical chemistry control reagent and which is suitable for storage in a dry state as attained by either lyophilization or spray drying methods.

The stability problems associated with such reagents has not however gone unrecognized and numerous attempts have been made to reduce these problems. Typically, however, these conventional methods have been limited to a very limited number of constituent (parameter) containing controls. Further, as is the case with hemoglobin controls, these single constituents have usually been inherently stable. See Bonderman et al., "A Lyophilized Hemoglobin Control Prepared From Stroma-free Hemolysates", Clin. Chem. 26/2:305-308 (1980); U.S. Patent No. 2,433,299 to Segers; and Proksch et al., "Preparation of Lyophilized Abnormal Hemoglobin Controls For A Cellulose Acetate Electrophoresis", A.J.C.P. 74/1:64-67 (1980).

Other attempts at obtaining increased stability have relied on surfactants such as Triton X-100, Proksch et al., "An Optically Clear Hypercholesterolemic Hypertriglyceridemic Quality Control Material Prepared from Animal Lipid Sources", Clinical Chemistry, Vol. 27/3:468-471 (1981) or complex mixtures of mucilagenous gums, polymers containing hydroxy groups, hydroxy alkylamine buffers, ethylene diamine tetraacetic acid, bovine serum albumin, polyvalent anion salts, or

-4-

sulfhydryl compounds as taught by Deutsch in U.S. Patent Nos. 3,413,198; 3,527,331 and 3,539,450.

It is an object of the present invention to obtain superior stabilizing results by employing less complex stabilizing means.

Muset in U.S. Patent No. 3,133,001 discloses the stabilization of a select group of enzymes: catalase, aldolase, and 17-hydroxy dehydrogenase, by the addition of sucrose, lactose or maltose at or near their saturation levels. Bonderman et al. claim stabilization of glucose-6-phosphate dehydrogenase by the addition of sucrose and lactose in an article in Clinical Chemistry Vol. 25/5: 815-816 (1977) entitled "Lyophilized Control for Determination of Glucose-6-phosphate Dehydrogenase Activity in Hemolysates".

It is still another object of the present invention to avoid adding stabilizing agents in concentrations which provide substantial, deleterious effects on other parameters normally found within a multiparameter clinical chemistry control reagent.

Mutti et al. in U.S. Patent No. 4,127,502 attempt to improve optical clarities of serum derived compositions by the addition of various sugars including mannitol and lactose. Table II of that patent indicates, however, that mannitol and lactose in fact failed to measurably reduce the optical density of the compositions. Heimburger et al. in U.S. Patent No. 4,056,484 disclosed the stabilization of a coagulation control by the addition of a protective gas containing at least 5% carbon dioxide in addition to various combinations of lactose saccharose or lactose.

-5-

It is a yet further object of the present invention to provide the correct methods for employing certain of the aforementioned sugars as stabilizing agents in multiparameter control reagents to obtain substantially increased stability and optical clarity.

Isoenzymes, or isozymes, as they are alternatively referred to, are enzymes in multiple forms which are capable of performing the same general function but at different rates. They are sufficiently different in chemical composition so that they are generally separable electrophoretically. One such isoenzyme, lactate dehydrogenase (LDH) is found in five electrophoretically distinct fractions. Each of these electrophoretic species of LDH is a tetramer consisting of two polypeptide chain units, H and M, present in different proportions: $H_4$, $MH_3$, $M_2H_2$, $HM_3$, and $M_4$. These five isoenzymes differ in catalytic activity (affinity for the substrate, pyruvate as measured by the Michaelis constant), amino acid composition, heat lability, and immunological responses. The two peptides H and M are coded by different genes. Thus the type of enzyme present is under genetic control and regulated by the conditions of the environment imposed upon the cell.

Similarly, creatinine kinase (CK) is another isoenzyme which contains subunits of either M's or B's and thus may be present as MM, BB, or MB. The MB form is clinically significant as an indicator of myocardial infarction. However, this form is unstable and tends to disassociate to reform the MM or BB types. It is another object of the present invention to stabilize a control reagent having the MB form.

The various proportions or combinations of isoenzymes present in the tissue may be related to the specific requirements of the cell in question and is thus affected by such factors as the extent of differentiation and development of the cell, as well as the level and type of metabolism occurring within the cell. Accordingly, the distribution between the various forms of isoenzymes provides diagnostically significant data. For instance, LDH exhibits significant control over cellular glycolosis. Specifically, $MH_3$ and $H_4$ isoenzyme types predominant in tissues with purely aerobic or respiratory metabolism. Accordingly, they may be used as diagnostic tools in determining the condition of muscles such as the heart, particularly with CK isoenzymes, the brain, liver and other organs in the case of alanine aminotransferase (ALT) or aspartase aminotransferase (AST). Yet another isoenzyme of significance is alkaline phosphatase gamma-glutamyl transpeptidase.

With such attention being placed on the determination of isoenzyme levels, particularly important in the case of cardiac critical care patients, it is axiomatic that adequate control reagents must be available in order to ensure the proper operation of manual and automated methods designed to determine these levels. Heretofore, such control reagents as were available, have been typically unstable due to the highly unstable nature of the enzymes themselves.

It is another object of the present invention to provide control reagents having the necessary levels of isoenzymes present therein in a stabilized format.

It is another object of the present invention to provide methods whereby isoenzyme control reagents may be stabilized.

Hemostasis is a complicated life saving procedure involving blood vessels, plasma, platelets, vessel walls, neural and humoral activity, all for the purpose of stanching bleeding. Although there has been much research, especially within the last few decades, concerning the process of coagulation specifically and hemostasis in general, there is much that is not completely understood particularly with regard to the specific details concerning the interaction of contributing mechanisms. For instance, it is now known that there are no less than 35 compounds which take part in the formation of a firm blood clot. Ostensibly, blood coagulation is thus one of the more complicated chemical processes occurring within the body. In fact, there are so many separate components within the scheme of coagulation that the International Committee on Nomenclature for Blood Coagulation formed by the International Hematology Congress has adopted a series of names which have now come into general acceptance. The most common of these names (with generally employed alternatives) and factor designations include the
following:

| | | |
|---|---|---|
| Factor I | Fibrinogen | |
| Factor II | Prothrombin | |
| Factor III | Thromboplastin | |
| Factor IV | Calcium | |
| Factor V | Proaccelerin, labile factor | |
| Factor VI | (no longer used) | |
| Factor VII | Serum prothrombin conversion accelerator (SPCA), stable factor | |
| Factor VIII | Antihemophilic factor (AHF) | |
| Factor IX | Christmas factor, plasma thromboplastin component (PTC) | |
| Factor X | Stuart Factor, Stuart-Prower factor | |
| Factor XI | Plasma thromboplastin antecedent (PTA) | |
| Factor XII | Hageman factor | |
| Factor XIII | Fibrin stabilizing factor | |
| Profibrinolysin | Plasminogen | |
| Fibrinolysin | Plasmin | |

Diagnostic determination of the presence and quantitation
of various of the above factors is important as many
clinically relevant diseases associated with abnormal
clotting are associated therewith. For instance, the
absence of Factor VIII is the most frequent and serious
cause of genetically determined clotting defects such as
hemophilia A which has been recognized for over 2,000
years. A deficiency in plasma concentration of Factor IX
is generally associated with the genetically determined
defect responsible for hemophilia B. Hemorrhagic disease
results from deficiencies in Factors X and XI whereas
deficiencies in Factor XII induce longer clotting times but still permit eventual clotting. Diseases of the liver or Vitamin K deficiencies have also been associated with abnormal levels of clotting Factors II, VII, IX, and X as these are predominantly produced by the liver. Other causes of abnormal clotting may be related to reduced platelet counts, thrombocytopenia, often associated with abnormal levels of clotting Factors II, VII, IX, and X as these are predominantly produced by the liver. Other causes of abnormal clotting may be related to reduced platelet counts, thrombocytopenia, often associated with pernicious anemia, certain drug therapies, irradiation or increased peripheral destruction by antibodies.

The clinical value of testing for coagulation times is not limited to the detection of genetic or pathological disease states but is also useful in the regulation of anticoagulant therapy. Anticoagulants typically inhibit the coagulation mechanism such as by the heparin mediated inhibition of Factor X by Antithrombin III. Deficiencies in the clotting mechanisms due to congenital defects, pathological conditions, or anticoagulation therapy have been discussed generally in Chapter 7, "Coagulation and Hemostasis" by Robert D. Langdell, In Clinical Diagnosis, Davidson and Henry, 1979.

As commonly understood, coagulation may occur by two pathways, the so called intrinsic pathway and extrinsic pathway. The former is generally triggered by the presence of a surface (thought to activate Factor XII) and, with the presence of phospholipids and calcium, through a number of steps eventually stimulates the formation of a stabilized fibrin clot. The partial thromboplastin time (PTT) test typically measures the intrinsic pathway wherein most congenital deficiencies occur. Consequently this type of test serves as an excellent presurgical coagulation screening test; however, since the reagent employed in this test is typically a platelet substitute, the PTT test does not measure platelet activity.

The extrinsic pathway is generally triggered by injury to tissue and the resultant exudation of tissue thromboplastin acts on Factors VII and X which, following a series of steps, thereafter forms a fibrin clot. The extrinsic pathway is generally tested by the so called one-stage prothrombin time test to thereby detect most of the factors depressed by oral anticoagulant drugs for anticoagulant therapy control. The thrombin time test typically measures the quantity and reactivity of fibrinogen. This test is a rapid, semiquantitative test and is consequently the test of choice for intravascular clotting and fibrinolysis analysis. The above is presented in review in accompanying Table I as well as a booklet entitled "Concentric Concepts of Coagulation 1971, 1975" available from Ortho Diagnostics, Inc., Raritan, New Jersey.

### Table 1

INTRINSIC PATHWAY
TRIGGER:  SURFACE

EXTRINSIC PATHWAY
TRIGGER:  TISSUE INJURY

Platelets
↓
XII      +Phospholipids
↓

III Tissue Thromboplastin

[Partial
Thromboplastin
Time (PTT)]

XI       +Ca

VII     (One-stage Prothombin
              Time)

└→ IX

└→ VIII

└→ X  <─────────────

Fibrinogen

V ──→ II ──→ Thrombin ───→ I ──→ Fibrin ──→ XIII ──→ Stabilized
                                              Clot              Fibrin Clot

Prothrombin

(Thrombin Time Test)

Roman Numerals indicate Factor designations.

With such attention being placed on the determination of a particular patient's coagulating ability and the presence or absence of specific factors, it is axiomatic that adequate control reagents must be available in order to ensure the proper operation of manual and automated methods designed to determine these factor levels and coagulation in general. Heretofore, such control reagents as were available have been typically characterized by relatively short shelf lifes both in the liquid and dry state due largely, in part, to the unstable nature of the ingredients or by their inability to withstand drying procedures such as lyophilization.

It is another object of the present invention to provide plasma-renin and coagulation control reagents having the appropriate levels of particular factors present therein in a stabilized format and capable of being lyophilized.

It is another object of the present invention to provide methods whereby coagulation control and plasma-renin control reagents may be stabilized.

It is another related object of the present invention to provide reagents in a stabilized format useful in manual and automated renal hypertensive testing and coagulation testing.

-13-

Steroid receptor analyses are performed in the clinical environment with increasing frequency due, at least, in part to the heightened awareness for the diagnostic value of the results. Specifically, it is now becoming generally accepted that an assessment of the presence of steroid receptors in biopsies of human breast-carcinomas is essential to enable correct selection of hormone therapy. Although the mechanisms regarding interaction between receptors and therapy is as yet undetermined, it does seem clear that both estrogen and progesterone receptors may be utilized as predictive indices of a breast cancer patient's response to hormonal manipulation. Indeed, it is a commonly accepted principle that the presence of both receptors enhances the effectiveness of steroid therapy and makes such a route of treatment a viable alternative vis-a-vis chemotherapy or surgery. The chart presented below characterizes the present view regarding the presence or absence of estrogen receptors (ER) and/or progesterone receptors (PgR):

Diagnosis of Advanced Breast Cancer

Test Results:    $ER^+/PgR^+$         $Er^+/PgR^+$         $Er^-/PgR^-$

Treatment:  Endocrine    Combined   Anti-      Chemotherapy
            Therapy      Chemo-     Estrogen   of
                         Endocrine  Therapy    Surgery
                         Therapy

A general but informative discussion is provided by Wittliff, et al., Physiopathology of Endocrine Diseases and Mechanisms of Hormone Action, pages 397-411, 1981, Alan R. Liss, Inc., New York, New York, in a chapter entitled "Methods of Steroid Receptor Analyses and Their Quality Control in the Clinical Laboratory."

The progress in developing ER and PgR tests to clinical significance has been impeded by a variety of factors acting in concert. Foremost of those is the basic instability of steroid receptors due to their heat labile nature. This is of particular concern with control reagents necessary for comparison with any tissue biopsy. Consequently, the conventional art has sought a variety of mechanisms in an attempt to ameliorate the stability problem.

Relatively early it was discovered that the progesterone and estrogen receptors were relatively stable while in the form of tissue preparations, particularly when such preparations were lyophilized and kept frozen. See for instance Koenders, et al., "Influence of Lyophilization and Subsequent Storage of Target Tissue on Steroid

0130708

-15-

Receptors, Steroid Receptors and Hormone-Dependent Neoplasia" edited by Wittliff, Masson Publishing, New York, New York. Such a solid tissue control material is comparable to patient biopsy tissue and accordingly must be treated in like fashion. Some investigators advocate the tissue control's merit as providing a control for the homogenization aspects of the assay procedure. To date, only one tissue control material has been available (from New England Nuclear under the tradename Rianen Assay System) and it has failed to relieve the existing interlaboratory confusion resulting from the plethora of distinguishable techniques and methods being practised. As expected, this nonuniformity of procedures effectively prohibits useful correlation of interlaboratory results.

The lack of common procedures has been further exacerbated because some investigators have sought to maintain a single laboratory control by making their own progesterone or estrogen receptor controls, typically from rat uteri. As may be expected, clinical environments do not lend themselves to the efficient production of control reagents especially since production of these materials is a difficult, time consuming and complex matter. Devotion of valuable resources to the production of control reagents not only reduces the clinical laboratory's effectiveness, but also introduces significant sources of error further limiting the ability to compare results from different laboratories.

It is another object of the present invention to provide a control material possessing both estrogen and progesterone receptors which may be commonly used by laboratories as a control reagent thereby instituting a standard permitting the comparison of laboratory results.

Materials previously employed to provide estrogen receptors include human breast tumor and calf uterus tissue.

-16-

It has been reported that when such tissue is lyophilized and kept at 0-4°C, there may be no loss of estrogen binding sites for up to two years. See Benraad, et al., "Estradiol Receptor Activity in Lyophilized Calf Uterus and Human Breast Tumor Tissue", Cancer 46:2762-2764, 1980. It may be noted, however, that the material described by Benraad provides only estrogen binding receptor sites and makes no mention of progesterone binding sites. Progesterone sites have been traditionally characterized as even more unstable than estrogen sites thus greatly increasing the difficulty of providing a stable reagent material having both types of binding sites.

It is another object of the present invention to overcome these problems by providing a material having both progesterone and estrogen receptors in a form having the stability necessary for acceptance in the clinical environment.

Still other sources may be used to provide receptor binding sites. See for instance Korenman, "Radio-Ligand Binding Assay of Specific Estrogens Using a Soluble Uterine Macromolecule", J.Clin.End. and Med. 28:127-130, 1968 which describes the use of uteri from pregnant rats. Bojar, et al., "Investigation of the Thermostability of Steroid Hormone Receptors in Lyophilized Calf Uterine Tissue Powder", Cancer 46:2770-2774, 1980 provides discussion with respect to calf uterine tissue.

The actual structure of the receptors is still a subject for investigation and some recent theories are described by Wittliff in "Steroid Receptor Interactions in Human Breast Carcinoma", Cancer 46,12:2953-2960 (1980). Additional experimental results describing the ability of vanadate and sodium molybdate to inhibit the receptor activation process and thus preserve receptor activity necessary for subsequent assay testing, have been reported in a series of articles: Nishigori, et al., "Inhibition of Progesterone Receptor Activation by Vanadate," Archives of Biochem and Biophysics, Volume 203, 2:600-604, 1980; Anderson, et al., "Sodium Molybdate Increases the Amount of Progesterone and Estrogen Receptor Detected in Certain Human Breast Cancer Cytosols," Steroids Volume 35, 3:273-280, 1980; and Maki, et al., "Alterations in Glucocorticoid Receptor Conformation by Molybdate," J. Biochem. 87, 6:1851-1854 (1980).

As has been previously mentioned, investigators in this field have searched for a useful, stabilized cytosol control preparation. Stabilized as used herein means there should be a recovery of at least 90% of the original receptor activity following storage. A cytosol type of preparation, as opposed to the well-known powders heretofore generally available, constitutes tissue which has been homogenized and centrifuged to form a cell-free solution. Although cytosol solutions may be readily prepared, heretofore, none of these cytosol solutions contained both progesterone and estrogen receptors in a stabilized format. For instance, the cytosol preparation described by Vermousek, et al., in "Stable Standard for Determination of Oestrogen Receptor," J.Clin.Chem.Biochem 19:865, 1981, failed to provide more than 20% of the original binding capacity following lyophilization.

It is another object of the instant invention to provide a cytosol preparation suitable for lyophilization and storage in that form for at least a year which, upon reconstitution, provides at least 90% of the original receptor activity, said receptors including both estrogen and progesterone receptors.

-18-

The inability of conventional methods to provide such a preparation is further exemplified in an article by Koenders, et al., "Preparation of Lyophilized Reference Samples for Quality Control of Steroid Receptor Measurements," The Ligand Review 3:22-39, 1981. That article describes the stability of lyophilized tissues with respect to estrogen and progesterone receptor activities and at 4° storage, good activity was reported after 14 months. Compare this however to the reported lyophilized cytosol of pig and calf uterine tissues during storage over a similar period of time. As expected, the more hardy ER binding sites remained relatively unchanged; however, PgR activity became virtually nonexistent after only one and a half months of lyophilization storage. Indeed, even lyophilized human breast tumor tissue showed significant decreases in activity after only a few months' storage.

It is yet a further object of the present invention to provide stabilization methods and preparations to permit stabilized storage of calf uterine cystols having both ER/PR binding sites.

-19-

## Summary of the Invention

According to the present invention there is provided a stabilized clinical control reagent comprising an aqueous solution of biological analyte and sufficient plexiform stabilizing means selected from glucose, sucrose, reducing monosaccharide sugars and reducing disaccharide sugars to obtain substantially increased stability over that obtained without such plexiform stabilizing means.

Preferably the amount of plexiform stabilizing means used will be from 2 to 10 final volume percent of the reagent.

In accordance with one aspect of the present invention, there are provided multiparameter control reagents having their inherent levels of analytes and other constituents ("control" as used herein) or having specified amounts of analytes added to a base material (a "standard" as used herein) in addition to plexiform stabilizing means selected from reducing monosaccharide and reducing disaccharide sugars, preferably present in the final volume concentration in the range of about 2%-8%. The preferred plexiform stabilizing means and the reducing sugars are maltose, mannitol, cellobiose and lactose, the latter most being the most preferred on the basis of efficacy and economy. If cost is an insubstantial factor, however, then the penultimate sugar of those listed above is the most preferred.

The thusly stabilized multiparameter reagent, whose source is a body fluid, may be advantageously adjusted by the addition or deletion of constituents so that the reagent contains those constituents, analytes, and protein levels desired in accordance with the product's purpose and end use.

In accordance with a second aspect of the present invention, isoenzyme control reagents are provided for creatinine kinase, lactate dehydrogenase, alanine aminotransferase and aspartase aminotransferase which are substantially stabilized by the addition of plexiform stabilizing means. The preferred plexiform stabilizing means is maltose, mannitol, cellobiose or lactose with the latter most being the most preferred. The plexiform stabilizing means is advantageously provided in a final concentration range of about 2% - 8% with the ideal concentration occurring at about 6%.

As a result, the isoenzyme control reagent products of the present invention may be made to act and behave in a more similar fashion as those of a patient and indeed, the instant reagents are capable of being run on any of the separation systems presently in use: column chromatography, and the immuno-based separation type systems.

In accordance with a third aspect of the present invention, coagulation control reagents and plasma renin control reagents are provided which are substantially stabilized by the addition of plexiform stabilizing means. In the case of coagulation control reagents, anticoagulation means is added to substantially defibrinated plasma, whereby clotting components are held intact and residual fibrin formation is inhibited. Additionally, plexiform stabilizing means, selected from : sucrose, reducing monosaccharide sugars and reducing disaccharide sugars, is added so that it is present in the final product preferably in the range of about 3%-10% volume percent. The preferred plexiform stabilizing means is maltose, mannitol, cellobiose, glucose or lactose with the lattermost being the most preferred. Additionally, and in accordance with the type of coagulation control reagent desired, a specific clotting factor or factors may be removed such as Factor VIII and/or Factor I.

The plasma renin control reagent of the present invention comprises selected plasma to which has been added anticoagulation means whereby clotting components are held substantially intact, and plexiform stabilizing means similar to that described for the coagulation control reagent. The anticoagulation means is ideally chosen from the group consisting of citrate, oxalate, EDTA (ethylene diamine tetraacetic acid), heparin, and any combination of the foregoing.

In accordance with a fourth aspect of the present invention there are provided stabilized reagents containing both estrogen receptors and progesterone receptors comprising processed tissue having both estrogen and progesterone receptors associated therewith, a suspending buffer which in the preferred embodiment is HEPES, sodium molybdate or vanadate as a binding site protector, dithiothreitol, additional protein in an inactive form, bovine serum albumin in a preferred embodiment, added in order to insure the final reagent meets a predetermined total protein level and, plexiform stabilizing means for stabilizing and protecting the reagent during storage. This plexiform stabilizing means provides necessary protection during lyophilization by functioning as a cryoprotective agent.

Indeed it has been surprisingly found by the inventors hereof that estrogen **and** progesterone receptor containing materials may be stabilized, contrary to prior beliefs, for extended periods of time particularly in a lyophilized format. The methods for such preservation include the addition of both a binding site activation inhibitor and the plexiform stabilizing means, advantageously chosen to be sodium molybdate and the reducing sugar lactose, respectively.

Preferably, the clinical control reagents will have substantially all water removed, such as by lyophilization, to assist in any long term storage and stability

Brief Description of the Drawings and Tables      .

Further understanding of the principles of the present invention may be had by reference to the Figures and Tables wherein:

FIGURE 1 shows a comparison of LDH units per liter of a 14 day liquid stability study in controls having various lactose concentrations; .

FIGURE 2 graphically depicts the differences in absorbance of reagents containing different lactose concentration solutions over time;

FIGURE 3 shows creatinine levels over time in reagents having various lactose concentrations;

Table 1 shows lyophilization losses for various analytes in normal and abnormal levels for controls and for the quality control stabilized product of the present invention;

Table 2 shows wet and dry interference studies in a control product with normal analyte levels;

Table 3 presents the same data as Table 2 but for products with abnormal levels;

Table 4 presents wet accelerated stability data for various constituents;

Table 5 presents data for wet accelerated stability studies for abnormal controls listing various constituents;

-24-

Table 6 presents reconstitution stability recovery data for normal controls;

Table 7 presents reconstitution stability recovery data for products with abnormal analyte levels; and

Table 8 represents the activity of a CK isoenzyme control reagent stored in aqueous form at 37°C.

MULTIPARAMETER CONTROL REAGENT

The multiparameter control reagents of this aspect of the present invention are useful for manual methods and automated methods in chemistry analysis and in particular may be used with multichannel chemistry analyzers such as the ACA™ from Du Pont or the SMAC™ from Technicon. The control reagent material is a patient-like sample characterized by a range of values for each constituent, enzyme and analyte found therein. The closer the control material simulates a patient's sample in providing all unknowns at their proper levels and in appearance (i.e., optical clarity and the like), the more useful it is. Similarly, the longer it presents such characteristics, i.e. the greater the time period it can hold the activity level for each constituent in a stable fashion, the more valuable the control material.

As has been previously intimated, there has heretofore been great difficulty in stabilizing such a multiparameter product and in particular, stabilizing various analytes such as CPK, LDH, $CO_2$, bilirubin, among others without deleteriously affecting other constituents or the tests therefor. With the addition of the plexiform stabilizing means of the present invention, these analytes or constituents have now been stabilized and may be maintained in solution for at least ten days. In dry form, i.e. substantially all water has been removed, this stability period is greatly increased.

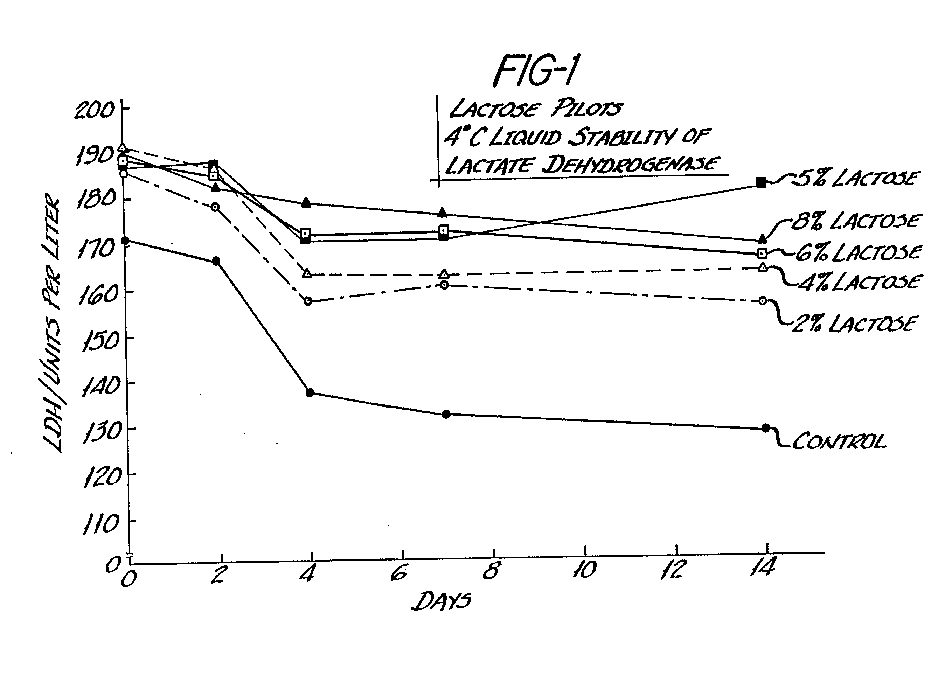

For instance, and with reference to Figure 1, lactate dehydrogenase (LDH), typically measured in units per liter, has heretofore generally been present in the initial solutions at approximately 190 units per liter. As may be apparent by reference to the control material (no plexiform stabilizing means added), typically a loss of approximately 20 units per liter was sustained upon lyophilization and reconstitution. Previously, such losses could not be avoided and it was hoped that they recurred on a consistent basis. Figure 1 shows the affects of the addition of lactose as a plexiform stabilizing means in various concentrations. As may be readily apparent, there is a significant increase in LDH stability over time.

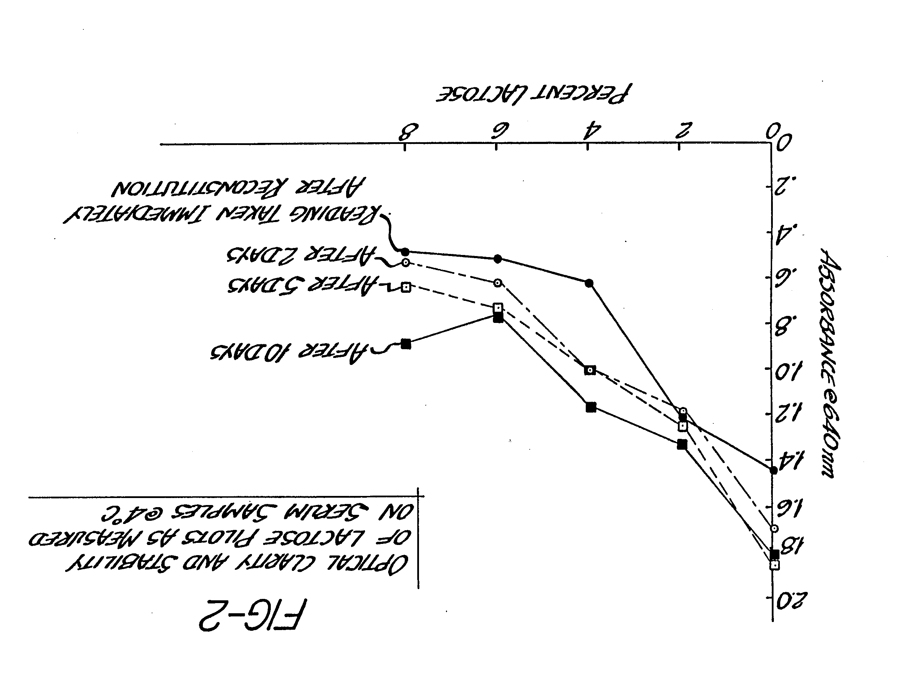

Additional surprising affects were encountered with the addition of lactose and include the reduced absorbance with increasing lactose concentrations. See Figure 2. In fact, it has been discovered that the reconstituted materials typically exhibit two or more times clearer optical density readings than without the addition of such plexiform stabilizing means.

However, and as may be seen by reviewing Figure 2, there is little absorbance gain with an increase of the concentration from 6% to 8% and indeed, as the percentages increase to 8% and beyond, other disadvantages become more apparent. For example, the high percentage levels become quite significant with respect to the limits of solubility and thus problems with solubilizing the sugar. Further, and as may be expected with the addition of sugar to any media, an increase in bacterial growth may be expected and this is only exacerbated with higher concentration levels. Accordingly, it may be desirable to add an antimicrobial substance in sufficient concentration to effectively

-26-

reduce or eliminate the possibility of microbial growth and resultant contamination. Such an antimicrobial should, of course, not interfere with any of the other constituents or with the testing methods. One preferred antimicrobial is chloramphenicol present, in a final concentration in the range of about 10 ppm to 1000 ppm. In this regard, it should be noted that the choice of plexiform stabilizing means will have a substantial affect on the types and amount of microbial growth to be encountered. For instance, sucrose is susceptible to fermenting bacteria and yeasts which are typically more difficult to control. Indeed, they are virtually impossible to eliminate by filtering due to the presence of spores, and other methods either destroy the product or are prohibitively expensive. Conversely, lactose fermenters are much more susceptible to antimicrobials and thus may be more easily controlled. Accordingly, lactose is greatly preferred over sucrose as a plexiform stabilizing means.

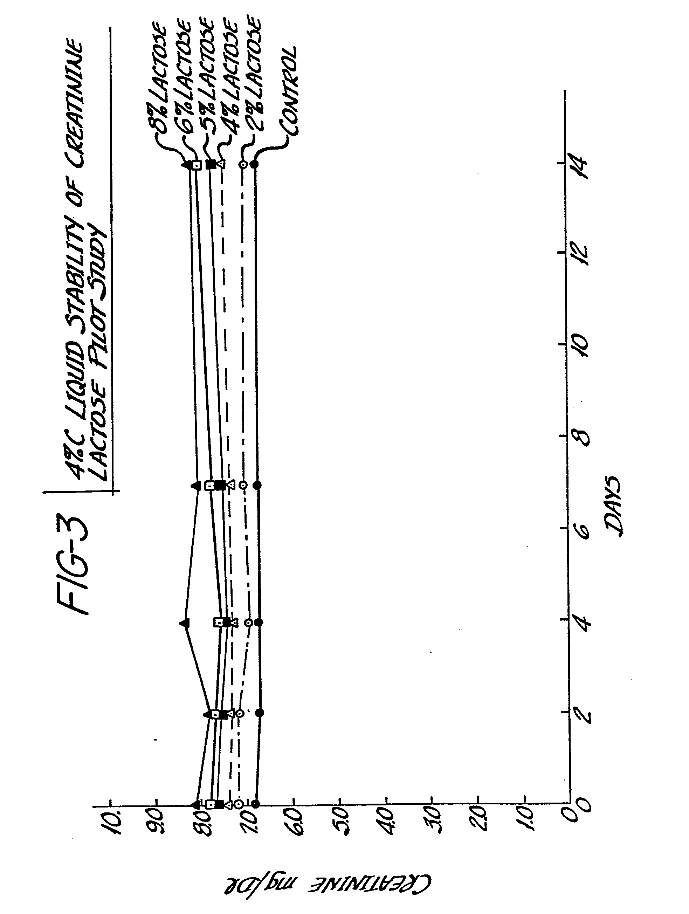

With the addition of the plexiform stabilizing means of the present invention, it has been noted that there is a positive bias affect on creatinine as shown in Figure 3. Indeed, with the addition of lactose in concentration ranges of approximately 4% to 8%, an increase in creatinine levels of approximately 1mg per deciliter results. Although this type of an interference is preferably to be avoided, it is not significantly deleterious as the positive bias merely puts the creatinine levels at the top of the normal range. Similarly, as may be expected whenever material is added, the osmolality increases as does total protein levels when tested by refractometers. Accordingly, and in order to limit these undesirable affects, the high concentration ranges and in particular those significantly in excess of 8%, are to be avoided. It is also noted that if sucrose is chosen as the plexiform stabilizing means, then any of the glucose oxidase procedures will in effect actually be

RD-50

measuring the amount of sucrose, an invert sugar, in addition to the levels previously present. Thus, glucose is a sugar to be preferably avoided.

The few, limited disadvantages incurred by addition of the plexiform stabilizing means of the present invention are, however, far outweighed by the numerous and significant advantages gained thereby. For instance, and in addition to the aforementioned stability increases, many constituents previously unstable or requiring augmentation, may now be measured over significant periods of time in their normal ranges without the addition of extraneous materials. For instance, many of the enzymes, such as CPK, incurred 50% or greater losses in the lyophilization step alone. These enzymes are now present in stable configuration and within normal levels even following lyophilization. Similarly, $CO_2$ is maintained in solution for significantly greater periods of time thus also limiting the otherwise concomitant rise in pH. In fact, $CO_2$ has been such a difficult constituent to measure heretofore, that many of the instruments presently available are incapable of detecting $CO_2$ with any great accuracy. It should be noted that bacterial growth and the like can result in the disadvantageous alteration of $CO_2$ levels.

Another unexpected surprise concerns the constituent bilirubin. This component is normally stable only at high pHs and accordingly, one would not expect stability at physiological pH (the pH of the control reagent; in vivo it is constantly being removed by the liver), however, with the addition of lactose, bilirubin becomes stabilized.

Still other unexpected results were discovered. As expected, with the addition of the plexiform stabilizing

-28-

means, the actual freezing point is depressed. Depression is generally associated with slower freezing rates. However, faster freezing of the product of the present invention has been observed. The inventors attribute this in part to the loss of the eutectic point plateau. In conjunction with this, it has been noted that without the addition of lactose, previous lyophilization methods on otherwise similar products resulted in a 2:1 ratio of powder versus crystalline cake configurations. The explanation of this phenomenon is as yet, totally unknown. With the addition of lactose, however, and the resultant faster freezing, it has been noted that all of the cakes are crystalline in nature and upon reconstitution, the absorbance measured on a vial to vial basis is far more uniform.

Although the inventors do not wish to be held to a theory, they have postulated that the plexiform stabilizing means of the present invention acts to provide a three dimensional structure which assists in holding the analytes in juxtaposition vis-a-vis one another while water is sublimed. Thus, water may be more quickly removed during lyophilization and more quickly reinserted into the structure during reconstitution. Thus, the plexiform stability means results in significant cold stability. Lyophilization with or without freeze concentration has been predominantly used to remove water as it operates at low temperatures, typically associated with decreased protein lability, disassociation, degradation and the like, with the plexiform stabilizing means of the present invention. However, like stability advantages are also associated with increased heat. Thus, the water may now also be removed with "hot" means (less than boiling) such as by spray drying techniques, and the like, commonly known .

Another important characteristic of the plexiform stabilizing means of the present invention is the associated reducing nature. In particular, the monosaccharide and disaccharide reducing sugars are preferred and of those, maltose, mannitol, cellobiose and lactose are employed in the preferred embodiments. If cost is not a factor regarding the constitution of the controls, then cellobiose is the most preferred reducing sugar as it subjectively produces the most advantageous results with limited or insignificant disadvantages or affects. Cellobiose is, however, an expensive commodity as compared to other preferred reducing sugars and in view of modern economic trends, lactose is employed in the most preferred embodiment as it closely parallels cellobiose in all categories except price. Indeed, the selection of the proper sugar for the plexiform stabilizing means is critical in order to obtain the advantages of a stabilized multiparameter control reagent. For instance, isomaltose provides some increased stabilization as well as many of the other advantages previously discussed. However, with this sugar, all saccarogenic amylase tests are eliminated. Typically, nonreducing sugars do not work well with respect to stability and accordingly, those sugars are to be preferably avoided.

Other surprising results include the increased reconstitution speeds which are obtained with plexiform stabilized multiparameter control reagents. Presumably, this may be a result of the three-dimensional structure imposed by the plexiform stabilizing means which not only assists water in resurrounding the constituents, but also increases the speed with which proteins unfold. The sugar probably also provides an environment which sterically hinders motion, particularly that of the larger molecules, and thus reduces degradation otherwise resulting therefrom. The above, however, represents theoretical conjecture by the inventor and is not to be construed as a limitation hereof.

Table 1 presents data depicting lyophilization losses, obtained by comparing a wet value against the value obtained of a reconstituted, lyophilized material. The control material did not have lactose added thereto while the QCS (quality control system) did have the plexiform stabilizing means, lactose, added thereto. The normal and abnormal columns represent materials having the normal range of constituents and elevated ranges of contituents respectively. As may be readily apparent, the lyophilization losses incurred with the QCS product are significantly less than those incurred in the control. It should be noted, however, that the AcP of 60% is within the accuracy limits of testing since at the normal levels, this only represents a reading difference of 1 unit. Thus, the 60% could just as easily have been 0%.

Tables 2 and 3 present data concerning interference studies for normal and abnormal multiparameter reagents. The QCS product had the plexiform stabilization means, lactose, added and the control product did not. The wet column presents values obtained prior to lyophilization while the dry column presents values obtained following reconstitution of a lyophilized material.

Tables 4 and 5 depict accelerated stability data for a lyophilized normal and for a lyophilized abnormal QCS product respectively. The control column presents the mean of measurements made at 0 time while the test sample columns represent the mean of samples measured following 26 days of storage at 37°C. This period represents the equivalent of approximately 3 years when stored at +5°C. Typically, variations around 100% may be attributable to the error ranges normally associated with readings of the particular constituent.

Similarly, Tables 6 and 7 represent reconstitution
stability recovery data for normal and abnormal lactose
stabilized multiparameter control reagents.

## TABLE 1

### Lyophilization Losses

| Constituent | Control | | QCS | |
|---|---|---|---|---|
| | Normal | Abnormal | Normal | Abnormal |
| AcP | 80% | 13% | 60% | 0 |
| AlP | 32% | 35% | 0 | 5% |
| Amy | 7% | 3% | 0 | 0 |
| GOT | 6% | 7% | 0 | 1% |
| GPT | 18% | 17% | 0 | 4% |
| CK | 47% | 23% | 0 | 1% |
| GGT | 0 | 0 | 0 | 0 |
| LDH | 4% | 4% | 0 | 0 |
| Lip | 0 | 18% | 0 | 0 |
| $CO_2$ | 52% | 64% | 28% | 35% |

## TABLE 2

### Normal Interference Studies

| | WET | | DRY | |
| --- | --- | --- | --- | --- |
| | QCS | Control | QCS | Control |
| Acid Phos | 1.0 | 1.0 | 0.4(1) | 0.2(1) |
| Alk Phos | 74 | 115 | 96 | 78 |
| Amylase | 50 | 55 | 52 | 51 |
| Bilirubin | .45 | .46 | 0.5 | 0.5 |
| BUN | 12.7 | 13.5 | 14 | 12.4 |
| Ca | 9.1 | 9.2 | 9.6 | 9.2 |
| Cholesterol | | | 161 | 134 |
| CK | 183 | 191 | 184 | 101 |
| Chloride | 102 | 107 | 111 | 106 |
| Albumin | 4.05 | 4.22 | 4.3 | 4.1 |
| Creatinine | 2.1 | 0.9 | 2.3 | 0.9 |
| Glucose | 90 | 92 | 93 | 86 |
| OT | 53 | 56 | 53 | 54 |
| PT | 28 | 27 | 27 | 22 |
| GT | 33 | 35 | 39 | 35 |
| Iron | 102 | 108 | 110 | 112 |
| LDH | 141 | 142 | 142 | 137 |
| Lipase | 24 | 20 | 17 | 17 |
| Mg | 2.1 | 2.5 | 2.3 | 2.5 |
| P | 3.4 | 3.2 | 3.6 | 3.2 |
| Sal | 9.6 | 9.6 | 10.0 | 9.8 |
| Triglycerides | | | 102 | 86 |
| TP | 6.3 | 6.0 | 6.6 | 5.9 |
| UA | 5.4 | 4.3 | 5.4 | 4.4 |
| Na | 134 | 137 | 143 | 135 |
| K | 4.2 | 4.3 | 4.6 | 4.2 |
| Li | 0.9 | 0.9 | 0.94 | 0.9 |
| $CO_2$ | 22 | 23 | 16 | 11 |
| $T_4$ | – | – | 83 | 80 |
| $T_3$ | – | – | 1.66 | 1.16 |
| $T_3U$ | – | – | 1.06 | 1.10 |
| Cortisol | – | – | 9.12 | 9.4 |
| Digoxin | – | – | 0.83 | 0.64 |
| TIBC | – | – | 328 | 313 |
| Phenylalanine | – | – | 1.9 | 1.8 |

1 - citric acid stabilizer tablet added in addition.

34

TABLE 3

Abnormal Interference Studies

| | WET | | DRY | |
|---|---|---|---|---|
| | QCS | Control | QCS | Control |
| Acid Phos | 11.0 | 11.2 | 11.0 | 8.6 |
| Alk Phos | 260 | 265 | 211 | 171 |
| Amylase | 392 | 386 | 400 | 376 |
| Bilirubin | 6.5 | 6.6 | 6.7 | 6.5 |
| BUN | 50 | 51 | 53 | 47 |
| Ca | 12.5 | 12.7 | 13.2 | 12.5 |
| Cholesterol | 123 | 108 | 124 | 93 |
| CK | 740 | 753 | 733 | 583 |
| Chloride | 116 | 116 | 120 | 116 |
| Albumin | 3.1 | 3.1 | 3.2 | 3.1 |
| Creatinine | 9.6 | 8.5 | 10.4 | 8.3 |
| Glucose | 310 | 312 | 324 | 306 |
| OT | 428 | 442 | 424 | 412 |
| HBD | | | | |
| PT | 118 | 123 | 113 | 102 |
| GGT | 111 | 105 | 115 | 106 |
| Iron | 237 | 243 | 259 | 244 |
| LDH | 445 | 471 | 475 | 451 |
| Lipase | 69 | 74 | 68 | 61 |
| Mg | 4.5 | 4.9 | 4.7 | 5.0 |
| P | 7.5 | 7.4 | 8.2 | 7.4 |
| Sal | 26 | 26 | 27.1 | 26 |
| Triglycerides | 70 | 54 | 82 | 63 |
| TP | 4.9 | 4.4 | 5.1 | 4.3 |
| UA | 9.5 | 8.3 | 10 | 8.3 |
| Na | 147 | 148 | 160 | 150 |
| K | 7.15 | 7.10 | 7.2 | 6.8 |
| Li | 2.4 | 2.4 | 2.46 | 2.5 |
| $CO_2$ | 17 | 17 | 11 | 6.2 |
| $T_4$ | | | 206 | 180 |
| $T_3$ | | | 3.95 | 3.3 |
| $T_3U$ | | | 1.92 | 1.98 |
| Cortisol | | | 56.4 | 47.2 |
| Digoxin | | | 2.61 | 2.2 |
| TIBC | | | 247 | 234 |
| Phenylalanine | | | 7.1 | 6.6 |

-35-

## TABLE 4

### ACCELERATED STABILITY DATA SHEET

Lot Number: 99A0216RD-2 (NORMAL)   Test samples stored at: 37° C. for 26 days

| Constituent | -25°C Control $\bar{x}$ | Test Samples $\bar{x}$ | % Recovery $\overline{x}T/\overline{x}C \times 100$ |
|---|---|---|---|
| Total Protein g/dl | 6.52 | 6.56 | 100.6% |
| Albumin g/dl | 4.51 | 4.43 | 98% |
| Bilirubin (Direct) mg/dl | 0.10 | 0.10 | 100% |
| Bilirubin (Total) mg/dl | 0.54 | 0.55 | 102% |
| Calcium mg/dl | 9.6 | 9.6 | 100% |
| Chloride mEq/L | 105 | 105 | 100% |
| $CO_2$ mEq/L | 16.5 | 14.6 | 89% |
| Creatinine mg/dl | 2.4 | 2.4 | 100% |
| Glucose mg/dl | 96 | 80 | 94% |
| Magnesium mg/dl | 2.2 | 2.1 | 95.5% |
| Phosphorus mg/dl | 3.47 | 3.50 | 101% |
| Potassium mEq/L | 4.81 | 4.66 | 97% |
| Sodium mEq/L | 143.6 | 143.7 | 100% |
| Urea Nitrogen mg/dl | 14.5 | 14.4 | 99% |
| Uric Acid mg/dl | 5.6 | 5.6 | 100% |
| Acid Phosphatase IU/L | 0.70 | 0.65 | 93% |
| Alk. Phosphatase IU/L | 96 | 85 | 88.5% |
| Amylase IU/L | 51 | 49 | 96% |
| Creatine Kinase IU/L | 204 | 189 | 93% |

-36-

<u>TABLE 4</u> (Continued)

<u>ACCELERATED STABILITY DATA SHEET</u>

Lot Number: 99A0216RD-2 (NORMAL)   Test samples stored at: 37° C. for 26 days

| Constituent | -25°C Control $\overline{x}$ | Test Samples $\overline{x}$ | % Recovery $\overline{x}T/\overline{x}C \times 100$ |
|---|---|---|---|
| GT IU/L | 36 | 35 | 97% |
| Alpha HBD IU/L | 247 | 250 | 101% |
| LDH IU/L | 146 | 137 | 94% |
| Lipase IU/L | 12 | 11 | 92% |
| AST IU/L | 53 | 50 | 94% |
| ALT IU/L | 27 | 24 | 89% |
| Cholesterol mg/dl | 165 | 165 | 100% |
| Triglycerides mg/dl | 104 | 103 | 99% |
| Total Lipids | 890 | 896 | 101% |
| $T_4$ mg/dl | 8.38 | 8.5 | 101% |
| $T_3$ ng/ml | 1.44 | 1.69 | 117% |
| $T_3$ Uptake % | 38.0 | 38.4 | 101% |
| Cortisol ug/dl | 10.50 | 10.37 | 99% |
| Digoxin ng/ml | 0.99 | 0.92 | 93% |
| Iron ug/dl | 113 | 115 | 102% |
| TIBC ug/dl | 350 | 397 | 113% |
| Lithium mEq/L | 0.99 | 0.98 | 99% |
| Phenylalanine mg/dl | | | |
| Salicylate mg/dl | 9.9 | 9.8 | 99% |
| Protein Electro- phoresis Albumin | 6.60 | 6.45 | 98% |
| Alpha 1 | 0.39 | 0.39 | 100% |
| Alpha 2 | 1.33 | 1.39 | 105% |
| Beta | 1.52 | 1.39 | 91% |
| Gamma | 1.56 | 1.79 | 115% |
| A/G Ratio | 1.375 | 1.302 | 95% |

-37-

## TABLE 5

### ACCELERATED STABILITY DATA SHEET

Lot Number: 99A0216RD-1(ABNORMAL)  Test samples stored at: 37°C for 26 days

| Constituent | -25°C Control[1] $\bar{x}$ | Test Samples $\bar{x}$ | % Recovery $xT/\bar{x}C \times 100$ |
|---|---|---|---|
| Total Protein g/dl | 5.04 | 5.08 | 101% |
| Albumin g/dl | 3.31 | 3.30 | 99.7% |
| Bilirubin (Direct) mg/dl | 0.31 | 0.36 | 116% |
| Bilirubin (Total) mg/dl | 6.80 | 6.77 | 99.6% |
| Calcium mg/dl | 13.1 | 13.1 | 100% |
| Chloride mEq/L | 114 | 115 | 101% |
| $CO_2$ mEq/L | 11.2 | 9.6 | 86% |
| Creatinine mg/dl | 10.4 | 10.5 | 101% |
| Glucose mg/dl | 324 | 315 | 97% |
| Magnesium mg/dl | 4.7 | 4.6 | 98% |
| Phosphorus mg/dl | 8.02 | 8.04 | 100% |
| Potassium mEq/L | 7.34 | 7.26 | 99% |
| Sodium mEq/L | 159.4 | 159.8 | 100% |
| Urea Nitrogen mg/dl | 53.1 | 53.7 | 101% |
| Uric Acid mg/dl | 9.9 | 9.8 | 99% |
| Acid Phosphatase IU/L | 11.20 | 11.13 | 99% |
| Alk. Phosphatase IU/L | 237 | 224 | 94.7% |
| Amylase IU/L | 412 | 406 | 98.5% |
| Creatine Kinase IU/L | 751 | 731 | 97% |
| GT IU/L | 117 | 115 | 98% |
| Alpha HBD IU/L | 542 | 554 | 102% |

1 - Control represents measurements at 0 time.

TABLE 5 (Continued)

ACCELERATED STABILITY DATA SHEET

Lot Number: 99A0216RD-1(ABNORMAL)   Test samples stored at: 37°C for 26 days

| Constituent | -25°C Control $\overline{x}$ | Test Samples $\overline{x}$ | % Recovery $xT/\overline{x}C \times 100$ |
|---|---|---|---|
| LDH IU/L | 482 | 470 | 97.5% |
| Lipase IU/L | 68 | 64 | 94% |
| AST IU/L | 424 | 394 | 93% |
| ALT IU/L | 116 | 103 | 89% |
| Cholesterol mg/dl | 122 | 121 | 99% |
| Triglycerides mg/dl | 78 | 80 | 103% |
| Total Lipids | 888 | 851 | 96% |
| $T_4$ mg/dl | 20.34 | 20.52 | 101% |
| $T_3$ ng/ml | 3.78 | 3.91 | 103% |
| $T_3$ Uptake % | 71.0 | 68.1 | 96% |
| Cortisol ug/dl | 56.02 | 54.90 | 98% |
| Digoxin ng/ml | 2.78 | 2.80 | 101% |
| Iron ug/dl | 252 | 253 | 100% |
| TIBC ug/dl | 266 | 308 | 116% |
| Lithium mEq/L | 2.60 | 2.60 | 100% |
| Salicylate mg/dl | 27.1 | 27.0 | 99.6% |

## TABLE 6

### RECONSTITUTED STABILITY RECOVERY DATA SUMMARY

$\overline{x}T$ = mean of test                    Lot #99A0216 Rd-2 (Normal)

%R = percent recovery = $\overline{x}T/\overline{x}C$ for Day 0 and

$\overline{x}$/Day 0 $\overline{x}T$ for other days

| DAY | 0 | 1 | 2 | 3 | 6 | 7 | 10 | 14 | 17 | 21 | |
|---|---|---|---|---|---|---|---|---|---|---|---|
| | $\overline{x}T$ | $\overline{x}T$ | $\overline{x}T$ | $\overline{x}T$ | $\overline{x}T$ | $\overline{x}T$ | $\overline{x}T$ | $\overline{x}T$ | $\overline{x}T$ | $\overline{x}T$ | %R |
| Total Protein g/dl | 6.61 | 6.60 | 6.63 | 6.63 | 6.63 | 6.61 | – | 6.47 | – | 6.50 | 98.3% |
| Albumin g/dl | 4.44 | – | – | – | – | 4.38 | – | 4.40 | – | 4.47 | 100.7% |
| Bilirubin(Dir) mg/dl | 0.09[1] | – | – | – | – | 0.10[2] | – | 0.16[2] | – | 0.15[2] | 166.7% |
| Bilirubin(Tot) mg/dl | 0.51 | 0.53 | 0.50 | 0.51 | 0.56 | 0.49 | – | 0.51 | – | 0.45 | 88.2% |
| Calcium g/dl | 9.6 | – | – | – | – | 9.7 | – | 9.6 | – | 9.7 | 101.0% |
| $CO_2$ mEq/L | 14.5 | – | – | – | – | 14.2 | – | 15.8 | – | 15.8 | 109.0% |
| Creatinine mg/dl | 2.4 | – | – | – | – | 2.3 | – | 2.3 | – | 2.3 | 95.8% |
| Glucose mg/dl | 93.5 | 94 | 93.5 | 94 | 94 | 94.5 | – | 95 | – | 94 | 100.5% |
| Magnesium mg/dl | 2.1 | – | – | – | – | 2.1 | – | 2.1 | – | 2.1 | 100.0% |

TABLE 6 (Continued)

| | | | | | | | | | | | |
|---|---|---|---|---|---|---|---|---|---|---|---|
| Phosphorus mg/dl | 3.66 | 3.63 | 3.71 | 3.61 | 3.74 | 3.69 | – | 3.72 | – | 3.70 | 101.1% |
| Potassium mEq/L | 4.48 | – | – | – | – | 4.70 | – | 4.64 | – | 4.45 | 99.3% |
| Sodium mEq/L | 144.2 | – | – | – | – | 140.9 | – | 141.6 | – | 141.7 | 98.3% |
| Urea Nitrogen mg/dl | 13.5 | – | – | – | – | 13.8 | – | 13.0 | – | 14.3 | 105.9% |
| Uric Acid mg/dl | 5.55 | – | – | – | – | 5.7 | – | 5.7 | – | 5.3 | 95.5% |
| Acid Phos.[4] IU/L | 0.34 | 0.40[5] | 0.67[6] | 0.75 | 0.89 | 0.81 | 0.88 | 0.36 | 0.42 | 0.21 | 61.8% |
| Alk. Phos. IU/L | 94.5 | 93 | 93.5 | 85 | 96 | 90 | 90 | 99 | 87 | 98 | 103.7% |
| Amylase IU/L | 54.5 | 52 | 52 | 54 | 55 | 54 | 48 | 48 | 49 | 49 | 89.9% |
| CK IU/L | 181.5 | 179 | 179.5 | .82.5 | 181 | 179.5 | 175 | 175 | 173 | 196[5] | 108.0% |
| GT IU/L | 37 | 47.5 | 33.5 | 35.5 | 33.5 | 32 | 39 | 35 | 36 | 35 | 94.6% |
| LDH IU/L | 145 | 141 | 137 | 134.5 | 137.5 | 133 | 127 | 133 | 128 | 127 | 87.6% |
| Lipase[3] IU/L | 20 | 12.5 | 14.5 | 15.0 | 20.5 | 16 | 15[5] | 9 | 11 | 10 | 50.0% |
| AST IU/L | 57 | 53.5 | 58 | 57.5 | 53.5 | 57 | 57 | 52 | 53 | 53 | 93.0% |

0130708

TABLE 6 (Continued)

| | | | | | | | | | | | |
|---|---|---|---|---|---|---|---|---|---|---|---|
| Cholesterol mg/dl | 163 | – | – | – | – | 163.5 | – | 161 | – | 167 | 102.5% |
| Triglycerides mg/dl | 107.5 | 103 | 102 | 92 | 102 | 105 | – | 103 | – | 103 | 95.8% |
| HBD u/l | 248 | 238 | 245 | 236 | 261 | 233 | 236 | 212 | 227 | 216 | 87.1% |
| ALT | 24.5 | 26 | 29 | 29 | 25.5 | 26 | 28 | 24 | 23 | 22 | 89.8% |
| Total lipids | 964 | – | – | – | – | 932 | – | 969 | – | 802 | 83.2% |
| $T_4$ ug/dl | 8.36 | – | – | – | – | 8.24 | – | 8.25 | – | 7.96 | 95.2% |
| $T_3$ ug/ml | 1.45 | – | – | – | – | 1.52 | – | 1.58 | – | 1.61 | 111.0% |
| $T_3$ Uptake % | 41.2 | – | – | – | – | 39.2 | – | 38.8 | – | 40.8 | 99.0% |
| Cortisol ng/dl | 10.36 | – | – | – | – | 10.56 | – | 9.89 | – | 10.08 | 97.3% |
| Digoxin ng/ml | 0.82 | – | – | – | – | 0.82 | – | 0.86 | – | 0.81 | 98.8% |
| Iron ug/dl | 112 | – | – | – | – | 112 | – | 111 | – | 114 | 101.8% |
| TIBC ug/dl | 336 | – | – | – | – | 332 | – | 336 | – | 349 | 103.9% |
| Lithium mEq/L | 0.96 | – | – | – | – | 0.97 | – | 0.96 | – | 0.95 | 99.0% |
| Phenylalanine mg/dl | 1.7 | – | – | – | – | 3.9 | – | 2.1 | – | 2.0 | 117.6% |

TABLE 6 (Continued)

| | | | | | | | | | | | |
|---|---|---|---|---|---|---|---|---|---|---|---|
| Salicylate mg/dl | 10.2 | - | - | - | - | 10.5 | - | 10.4 | - | 10.3 | 101.0% |
| Chloride mEq/L | 112 | - | - | - | - | 112 | - | 114 | - | 114 | 101.8% |

1 - determined via Am. Mon.

2 - determined via BMC.

3 - Although ACA readings are reported, this constituent is not accurately discernible on the ACA (Du Pont)

4 - These values are all within the limits of testing variation.

5 - New packaging

6 - Lot samples of and after this date did not have a citric acid tablet added.

-42-

0130708

## TABLE 7

### RECONSTITUTED STABILITY RECOVERY DATA SUMMARY

$\bar{x}T$ = mean of test

Lot #99A0216 RD-1   (Abnormal)

%R = percent recovery = $\bar{x}T/\bar{x}C$ for Day 0 and

$\bar{x}T$/Day 0 $\bar{x}T$ for other days

| DAY | 0 | 1 | 2 | 3 | 6 | 7 | 10 | 14 | 17 | 21 | |
|---|---|---|---|---|---|---|---|---|---|---|---|
| | $\bar{x}T$ | $\bar{x}T$ | $\bar{x}T$ | $\bar{x}T$ | $\bar{x}T$ | $\bar{x}T$ | $\bar{x}T$ | $\bar{x}T$ | $\bar{x}T$ | $\bar{x}T$ | %R |
| Total Protein g/dl | 5.13 | 5.12 | 5.14 | 5.13 | 5.13 | 5.15 | – | 5.06 | – | 5.06 | 98.6% |
| Albumin g/dl | 3.31 | – | – | – | – | 3.37 | – | 3.33 | – | 3.33 | 100% |
| Bilirubin (Dir) mg/dl | 0.17[1] | – | – | – | – | 0.41[2] | – | 0.49[2] | – | 0.34[2] | 200% |
| Bilirubin (Tot) mg/dl | 6.69 | 6.75 | 6.59 | 6.62 | 6.70 | 6.56 | – | 6.34 | – | 6.0 | 86.9% |
| Calcium g/dl | 13.3 | – | – | – | – | 13.4 | – | 13.1 | – | 13.4 | 100.8% |
| $CO_2$ mEq/L | 10.6 | – | – | – | – | 9.4 | – | 10.8 | – | 10.6 | 100% |
| Creatinine mg/dl | 10.5 | – | – | – | – | 10.5 | – | 10.5 | – | 10.5 | 100% |
| Glucose mg/dl | 328 | 324 | 323 | 325 | 325 | 322 | – | 329 | – | 328 | 100% |
| Magnesium mg/dl | 4.75 | – | – | – | – | 4.8 | – | 4.7 | – | 4.7 | 98.9% |

TABLE 7 (Continued)

| | | | | | | | | | | |
|---|---|---|---|---|---|---|---|---|---|---|
| Phosphorus mg/dl | 8.13 | 8.14 | 8.17 | 8.09 | 8.26 | 8.17 | - | 8.30 | - | 8.32 | 102.3% |
| Potassium mEq/L | 7.76 | - | - | - | - | 7.59 | - | 7.61 | - | 7.69 | 99.1% |
| Sodium mEq/L | 161.8 | - | - | - | - | 158.9 | - | 157.1 | - | 158.8 | 98% |
| Urea Nitrogen mg/dl | 52.5 | - | - | - | - | 53.9 | - | 51.5 | - | 53.8 | 102.5% |
| Uric Acid mg/dl | 10.1 | - | - | - | - | 9.9 | - | 10.1 | - | 9.8 | 97% |
| Acid Phos. IU/L | 11.24 | 11.01 | 10.92 | 11.36 | 10.99 | 11.54 | 11.39 | 10.80 | 11.06 | 10.01 | 89.1% |
| Alk. Phos. IU/L | 214 | 216 | 229 | 229.5 | 255.5 | 237 | 259 | 293 | 289 | 307 | 143% |
| Amylase IU/L | 403 | 406 | 402 | 399.5 | 404 | 410.5 | 411.5 | 411 | 411 | 406 | 100.7% |
| CK IU/L | 713 | 706 | 701 | 704 | 699 | 694 | 675 | 681 | 680 | 692 | 97.1% |
| GT IU/L | 117.5 | 120 | 113 | 115.5 | 115 | 114 | 112 | 113 | 114 | 114 | 97% |
| LDH IU/L | 478 | 476 | 464 | 447 | 459.5 | 453 | 432 | 440 | 431 | 426 | 89.1% |
| Lipase IU/L | 71.5 | 74.5 | 74.5 | 67.5 | 76.5 | 66 | 72 | 64 | 72 | 63 | 88.1% |
| AST IU/L | 431.5 | 432 | 427 | 436.5 | 436.5 | 432 | 436 | 425 | 426 | 427 | 99.0% |

-44-

0130708

TABLE 7 (Continued)

| | | | | | | | | | | | |
|---|---|---|---|---|---|---|---|---|---|---|---|
| Cholesterol mg/dl | 121 | – | – | – | – | 122 | – | 123 | – | 121 | 100% |
| Triglycerides mg/dl | 79 | 79 | 78 | 77 | 74.5 | 78 | – | 83 | – | 84 | 106% |
| HBD u/l | 562.5 | 539 | 540.5 | 560 | 510.5 | 520 | 508.5 | 486 | 481 | 486 | 86.5% |
| ALT u/l | 112 | 114 | 114 | 112 | 111 | 110 | 114 | 111 | 106 | 108 | 96.4% |
| Total lipids | 903 | – | – | – | – | 862 | – | 1007 | – | 786 | 87% |
| $T_4$ ug/dl | 19.15 | – | – | – | – | 21.23 | – | 20.76 | – | 20.61 | 105.6% |
| $T_3$ ng/dl | 3.27 | – | – | – | – | 3.67 | – | 3.64 | – | 3.64 | 111% |
| $T_3$ Uptake % | 71.2 | – | – | – | – | 73.6 | – | 74.4 | – | 74.0 | 103.9% |
| Cortisol ng/dl | 53.4 | – | – | – | – | 51.93 | – | 51.41 | – | 52.64 | 98.6% |
| Digoxin ng/ml | 2.63 | – | – | – | – | 2.59 | – | 2.90 | – | 2.85 | 108.4% |
| Iron ug/dl | 252 | – | – | – | – | 254 | – | 254 | – | 256 | 101.6% |
| TIBC ug/dl | 266 | – | – | – | – | 260 | – | 267 | – | 269 | 101.% |
| Lithium mEq/L | 2.54 | – | – | – | – | 2.68 | – | 2.64 | – | 2.58 | 101.6% |
| Phenylalanine mg/dl | 9.2 | – | – | – | – | 9.1 | – | 7.7 | – | 7.60 | |

TABLE 7 (Continued)

| | | | | | | | | | | | |
|---|---|---|---|---|---|---|---|---|---|---|---|
| Salicylate mg/dl | 27.1 | - | - | - | - | 28.1 | - | 28.1 | - | 28.7 | 105.9% |
| Chloride mEq/L | 125 | - | - | - | - | 123 | - | 126 | - | 127 | 101.6% |

1 - determined in Am. Mon.

2 - determined via BMC

3 - new package lot

-46-

0130708

It is to be understood that all of the above discussion is equally applicable to chemistry standard reagents which differ from chemistry controls in that levels or values of constituents in the former is known while in the latter, the constituents may be present anywhere within a given range of values or levels. It is to be further understood that stability as used herein shall mean later measured activity which is at least 90% of the original measured activity. It is also applicable to other types of clinically useful control reagents such as lipid and cholesterol controls, isoenzyme controls, and coagulation controls, and the like as shown below.

Stabilized Isoenzyme Control Reagents

As has been previously intimated, there has heretofore been great difficulty in stabilizing isoenzyme control reagents and in particular, stabilizing the various

-48-

isoenzyme subunits such as the CK isoenzyme unit MB without deleteriously affectinn other subunit components or the tests therefor. With the addition of the plexiform stabilizing means of the present invention, these isoenzymes and their subunit constituents have now been stabilized and may be maintained in solution for significantly greater periods of time than previously possible. In dry form, obtained when substantially all water has been removed such as by lyophilization, the stability period is increased to-an even greater extent.

The addition of the plexiform stabilizing means of the present invention results in depression of the actual freezing point. Depression of the freezing point is generally associated with slower freezing rates, however, faster freezing of the product of the present invention has been observed. It would appear, however, that the plexiform stabilizing means when added to the material of the present product results in the loss of the eutectic point plateau thus actually increasing the freezing rate. Associated with this phenomenon is the observation that the cakes formed during freezing are uniformly crystalline, as opposed to the often occurring powder forms. This would imply that the plexiform stabilizing means is holding the constituents in a stable, three dimensional "crystalline" structure thereby assisting in the removal of water, the stabilization of the constituents themselves, as well as speeding the reconstitution of the lyophilized material. These and other complex interactions are more fully described above with reference to the multiparameter-control.

In order to make the isoenzyme control reagents of the present invention, a specific human tissue was selected in accordance with the type of enzyme containing product to be produced. For instance, for the CK MB subunit, heart tissue is selected, for the CK MM enzyme subunit muscle tissue is employed while for the CK BB subunit brain tissue is used. Similarly, these and other tissues including non-human tissues may be used as the source material for the isoenzymes or their subunits in accordance with well-known knowledge.

The tissue is then treated by grinding etc. to a form suitable for isoenzyme isolation by either chromatography, electrophoresis or other immunologically based system in accordance with techniques well-known. The thusly isolated enzyme components may be added in diagnostically significant proportions to a preferably preclarified, human sera base. The human sera is advantageously clarified to remove lipids by either filtering, freezing, reconstituting and filtering or by the addition of silica compounds such as aerosil all of which are methods well-known.

It is then advantageous to add to this material, a stabilizing cofactor as may be necessary to assist in the maintenance of binding site activity. For instance, for the CK isoenzymes, sulfhydryl containing compounds such as glutathione or dithiotreotol or n-actyl-cysteine are useful. Further, the ideal product also includes chelating means for removing metallic ions, if present, which may interfere with enzyme activity. If the enzyme's activity is not affected by the presence of metallic ions, this agent need not be included. Similarly, if no metallic ions are present this agent may again be eliminated. An example of such a chelating agent is ethylenediamine tetraacetic acid (EDTA).

Lastly, the isoenzyme product of the presentwill preferably further comprise a weak, nonphosphate buffer. It is preferred that a nonphosphate buffer is employed in order to avoid phosphorus containing compounds which may

-50-

otherwise interfere with some of the reactions, particularly those involving the adenine triphosphate (ATP) cycles. The buffer should be "weak" i.e., within the range of about 10-200 millimolar concentration. The pH of the buffer should ideally be selected or adjusted in order to preferably optimize activity and maximize stability. For instance, with the CK and LDH isoenzymes, a pH of approximately 7 is advantageous while the ideal pH for the alkaline phosphatase isoenzymes is in the range of about 7.6 to 7.8.

Finally, plexiform stabilizing means is added in order to provide the stability and other aforementioned advantages described herein. Such a plexiform stabilizing means is selected from the group consisting of monosaccharide and disaccharide reducing sugars and preferably will be selected from the group consisting of mannitol, maltose, cellobiose and lactose. As described above the most preferred, in terms of stability advantages and economic considerations, is lactose. The inventors hereof have found it desirable to present the plexiform stabilizing means in a final concentration range of about 2%-8% with the most preferred embodiment comprising approximately 6%.

The plexiform stabilizing means not only retards the disassociation of isoenzymes whose subunits tend to disassociate rather easily, but it also stabilizes the isoenzyme's electrophoretic patterns. The concentration of the plexiform stabilizing means is selected to retain the enzyme activity even if the product is freeze dried and may be preferably optimized for maximum enzyme activity. If too little plexiform stabilizing means is incorporated into the final product, then insufficient protection is derived. However, it is undesirable to add too much plexiform stabilizing means which in high concentrations not only may precipitate out of solution but may also block the active site of the protein. Thus, high concentrations of the plexiform stabilizing means actually tend to reduce enzyme activity and are consequently avoided.

An example of increased stability is presented in accompanying Table 8. The data therein represents the activity of a CK isoenzyme control stored in aqueous form at 37°C. It compares the data observed with an unstabilized isoenzyme control against the stabilized isoenzyme control from day 0 to day 30. Still greater increases in stability may be expected at lower temperatures i.e., 5° storage as well as in lyophilized storage format.

–52–

## TABLE 8

### CK ISOENZYME CONTROL

### 37°C WET STABILITY STUDY

| DAY | UNSTABILIZED CK | STABILIZED CK |
|---|---|---|
| 0 | 984 | 945 |
|   | 984 | 931 |
| 3 | 944 | 945 |
|   | 944 | 956 |
| 5 | 981 | 918 |
|   | 972 | 930 |
| 9 | 938 | 901 |
|   | 942 | 672[1] |
| 12 | 961 | 942 |
|    | 965 | 948 |
| 16 | 695 | 679 |
|    | 702 | 670 |
| 30 | 403[2] | 466[2] |
|    | 408[2] | 462[2] |

[1] short sample

[2] 1:2 dilution results

-53-

## Stabilized Coagulation Control Reagents

The coagulation control reagents products of this
aspect of the present invention are useful for use in manual methods as well as automated methods in
chemistry analysis and are particulary useful for those
tests involving coagulation and plasma renin activity.
These tests include, for instance, renal hypertension
testing, actual coagulation testing, testing for
angiotension I and/or II, and renin activity testing

-54-

which is typically run only on fresh human plasma. As may be expected, particularly with the lattermost test, adequate control reagents are difficult to obtain particularly in a suitably stabilized format.

Such a stabilized format is particularly desirous in the clinical environment in order to minimize cost expenditures and to provide increased comparative integrity of the data on a daily testing basis.

Ideally, the coagulation control reagents will simulate a patient's sample as closely as possible except, to the extent the controls may be artificially altered in order to provide factor deficient controls to levels which may be reasonably expected with particular disease states. For instance, a patient suffering a cardiac infarction may be expected to have increased levels of renin. Renin is known to act on plasma proteins thereby forming angiotensin, in turn, responsible for an increase in blood pressure occasioned by constricted blood vessels. The renin, a proteolytic enzyme believed to be produced in the efferent glomerular arterioles, reacts with hypertensinogen to product angiotensin II. The latter is a decapeptide hormone which, in addition to influencing blood vessel constriction, also alters aldosterone secretion by the adrenal cortex. Angiotensin is also known as hypertensin. Aldosterone is a steroid hormone which function chiefly in regulating sodium and potassium metabolism and thereby it also plays an important part in cardiac function.

Accordingly, and in order to ensure accurate testing procedures, a plasma renin control reagent is a virtual necessity, and is particularly desirable if made in a stable format. In accordance with this aspect of the present invention, such a control reagent is now made

-55-

hereby possible. It comprises plasma, ideally from human origin, preferably selected for its high renin levels. Additionally, an anticoagulant is added whereby the clotting components may be held intact and fibrin formation inhibited. Such an anticoagulant may be selected from the group consisting of citrate, oxalate, EDTA, heparin or a combination thereof.

The desired stability is obtained by adding plexiform stabilizing means selected from the group consisting of reducing monosaccharide sugars and reducing disaccharide sugars. Preferred embodiments employ maltose, mannitol, cellobiose, lactose or glucose. The plexiform stabilizing means is added so that the final percent concentration is preferably in the range of about 3%-10%. As more fully described above in relation to the multiparameter control reagent, the plexiform stabilizing means is thought to provide a three dimensional "crystalline" structure whereby the proteins, analytes, and constituents of the control reagents are held in stabilized juxtaposition. It is further believed by the inventors hereof, although they do not wish such belief to be a limitation hereof, that the plexiform stabilizing means serves to hold the individual constituents in a substantially rigid manner thereby reducing the amount of degradation or lability which may be associated or occasioned by the physical movement of the analytes.

Further, the plexiform stabilizing means serves to assist in the sublimation of water during water removal processes such as by lyophilization. The actual mechanism by which this is accomplished is unknown. However, it has been observed, particularly with respect to the multi-

0130708

-56-

parameter control reagents . referred to above , that
reconstitution of the reagent after lyophilization is
accomplished with greater speed and with increased vial-
to-vial reproducability. These advantages may also be
expected following lyophilization of the control reagents
of this aspect of the present invention.

Similarly, the coagulation control reagents are ideally produced
from plasma having added thereto an anticoagulant such
as that described above, whereby the clotting components
are held intact and residual fibrin formation is
substantially inhibited. In addition to supplementation
with the above-mentioned plexiform stabilizing means, the
coagulation control reagent may be adjusted to remove
specific clotting factor or factors in accordance with the
type of coagulation control reagent desired. For
instance, should a reagent be desired which is substantially deficient in Factor I (fibrinogen) or Factor VIII
(antihaemophelic factor), then these components may be
removed such as by ethanol extraction or chilling,
respectively, or by other methods for other Factors as is
well-known in the art.

As with the plasma renin control reagent, the plexiform
stabilizing means acts to provide a three dimensional
structure to greatly increase stability, speed of
reconstitution following lyophilization and superior vial-
to-vial reproduceability. Further, and as is more fully
explained above , the speed with which
lyophilization is accomplished may actually be increased
despite the lowering of the freezing point. This is
thought to be generally due to the elimination of the
eutectic point plateau. Additionally, and for reasons not
yet fully explained, the reconstituted materials typically
exhibit greater optical clarity.

This is probably due, at least in part, to the decreased denaturation of proteins, particularly a problem during lyophilization.

Still other advantages are gained by the instant invention and include the observation that the defibrinated plasma appears more completely defibrinated with the addition of the plexiform stabilizing means than defibrinated plasma not so augmented. The observed, more consistent gel-like clot may thus be a natural progression therefrom and may also be indicative of the enhanced overall stability of other inherent clotting factors present in the plasma.

The most preferred reagents of this aspect of the present invention will also have substantially all water removed by processes well-known in the art. Such processes include, without limitation, lyophilization and the like, and serve to afford even greater shelf lives. Ideally, the resultant dry product is stored at approximately 2°- 8°C and reconstituted just prior to use.

Stability as used herein shall mean later measured activity which is at least 90% of the original measured activity.

Estrogen-Progesteone Control Reagents

A variety of target tissues containing both progesterone and estrogen receptors may be employed and include for instance animal tissues such as rat mammary glands, pig uteri and human tissues such as human breast material. The inventors hereof however have found that calf uteri, and in particular, the uterine endometrium, to be the most preferred material with which to work. This material does, however, require special handling. Ideally, the uterus should be obtained from a born calf, have a weight of approximately 30-50 grams, and be devoid of blood and connective tissue. After removal from the calf, the tissue is advantageously frozen at -80°C and subsequently processed under nitrogen. This includes breaking up the tissue into roughly one inch size pieces followed by grinding and pulverizing to obtain a fine powder. Thereafter the material is homogenized by either grinding or sonicating, processes well known, and the resultant material ultracentrifuged to remove cellular debris. The

-59-

supernatant is the cytosol fraction which is thereafter ideally diluted or otherwise adjusted to obtain the 2-4 milligram per ml protein levels desired in a final reagent. The protein level may be typically measured using well known standard protein assay methods. If necessary, protein such as Bovine Serum Albumin or preferably non-specific cellular protein such as globulin or the cytosol from mature bovine uteri may be added to obtain the required total protein levels.

The thusly processed material may be tested to determine the estrogen and progesterone binding capacity by employing a radiolabeled estrogen and progesterone such as that provided under the New England Nuclear Rainen trademark. Typically, six different concentrations of this radiolabelled material are utilized in the assay in conjunction with a nonspecific binding control for each concentration. The results are plotted and the x-intercept determined. Although the values (x-intercept) calculated cannot be classified on an absolute basis, due largely to the heretofore great variation between laboratory results, it is generally understood that ER in the range of 100 fmole ($10^{-15}$) per milligram of cytosol protein is interpreted as positive with the border line or gray zone ranges identified as 5-20 with 30+ fmole/mg identified as strongly suspicious. The progesterone receptor values run somewhat higher. 150 fmole/mg cytosol protein is understood to represent a positive result with the positive "gray zone" occurring at approximately 5-30 fmoles/mg cytosol protein.

The target tissue is preferably homogenized with a buffer such as TRIS or HEPES although the other "GOODS" buffer (see p. 396 of the Feb. 1983 SIGMA CATALOG) such as MOPS, MES, PIPES, etc. may be substituted therefor. Each of these buffers may be used at a 10 mmole/l concentration with a final concentration at reconstitution ideally

-60-

chosen to be in the range of about 0.025 molar to about 0.15 molar; the most preferred concentration being 50 mmolar. The most preferred embodiment comprises HEPES buffer as the inventors hereof have discovered the TRIS buffer has minimal buffering capacity at the preferred pH value of about 7.4 at the standard storage temperature of 4°C. It may be readily appreciated, however, that other buffers may be employed in substitution.

The preferred embodiment includes dithiothreitol in a final concentration in the range of 0.5-2 mmolar. It is believed that dithiothreitol protects the receptor sulfhydryl groups although the inventors hereof do not wish to be held to this theory. The preferred final concentration of this reagent otherwise known as Clelands reagent, should be in the range of approximately 1 mmole. Alternately, active sulfhydryl compounds such as monothioglycerol, dithioerythreitol, n-acetyl cystene, B-mercapto ethanol and the like may be substituted for dithiothreitol.

An important ingredient in this aspect of the invention is the addition of a binding site activation inhibitor such as sodium molybdate or vanadate. It is preferred that the inhibitor be provided in the range of approximately 1-5 mmolar final concentration, with the most preferred embodiment utilizing this reagent in a final concentration of about 2.5 mmolar. It is important to note that the amounts employed herein are significantly less than those employed by conventional techniques (10-20 mmole). The inventors hereof have discovered that the greater concentration ranges of the prior art interfere with the WADDELL type protein assays; an assay commonly employed by many researchers. As has been previously intimated, the mechanisms by which the activation inhibitors protect the binding sites is still relatively unknown.

-61-

All embodiments of the ER/PgR reagents contain an effective amount of a plexiform stabilizing means. The effective amount is that required to obtain significantly greater stability of the estrogen and progesterone receptors and is ideally in the range of 0 .05-0.4 final molar concentration. The inventors hereof have found reducing sugars to be the preferred plexiform stabilizing means as it is believed they serve to provide a 3 dimensional network for holding the binding site molecules and thereby protecting them from oxidation and stabilizing them during the various phases of lyophilization. These and related aspects are more fully described above.

The most preferred embodiments employ either sucrose, glucose, lactose or combinations thereof. Lactose is the most preferred. The ideal final concentration is approximately 75 mmolar.

The most preferred embodiment of the reagent will be lyophilized and will also include EDTA as a preservative preferably in a final concentration of approximately 0.1 mmolar. Such a preservative acts to inhibit microbial contamination; however, its absence results in no significant ER/PR assay differences. Other non-interfering preservatives may be employed in substitution.

The following example is given to illustrate this aspect of the present invention.

Calf uterine endometrium was separated from blood and connective tissue, frozen at - 80°C, and subsequently processed under nitrogen.  The frozen tissue was broken into roughly one inch size pieces and was ground and pulverised to obtain a fine powder.

The fine powder was suspended in 10mM HEPES buffer, pH 7.4, and homogenised by sonication.  The homogenised suspension was ultra centrifuged to remove cellular debris.

Mature bovine uterine cytosol, sodium molybdate, dithiothreitol, lactose and EDTA were added to the suspension, which was then lyophilised by standard procedures to produce the lyophilised reagent.

-63-

The lyophilised reagent was reconstituted by the addition of an aliquot of water. The size of the aliquot of water and the amounts of the agents added to the suspension were selected so that the reconstituted reagent had the following composition.

| | |
|---|---|
| Total protein | 2 to 4 mg/ml |
| Sodium molybdate (binding site protector) | 2.5 mM |
| Dithiothreitol (cofactor means) | 1mM |
| Lactose (plexiform stabilizing means) | 75mM |
| EDTA | 0.2mM. |

A sample of the lyophilised reagent was placed in a vial and kept for one year at 4°C. On reconstitution by addition of an aliquot of water, it was found that it had retained at least 90% of the tissue's receptor activity.

As may be readily appreciated by those skilled in the art various alterations and substitutions of the aforementioned constituents may be made without deviation from the spirit and scope of the instant invention. In particular, it will be appreciated that many of the optional constituents, such as preservative means, which are mentioned in respect of one aspect of the invention may also be used as desired, in other aspects of the invention. It will also be appreciated that any of the reagents may be supplied as a two vial kit, the first vial containing water-free, preferably lyophilised, reagent, and the second vial containing a suitable quantity of liquid for reconstituting the contents of the first vial.

-64-

CLAIMS

1.    A stabilized clinical control reagent comprising an aqueous solution of biological analyte and sufficient plexiform stabilizing means selected from glucose, sucrose, reducing monosaccharide sugars and reducing disaccharide sugars to obtain substantially increased analyte stability over that obtained without such plexiform stabilizing means.

2.    The reagent of claim 1, containing from 2 to 10 final volume percent of said plexiform stabilizing means.

3.    The reagent of claim 1 or claim 2, having substantially all the water removed therefrom.

4.    The reagent of claim 3, wherein the water was removed by lyophilisation.

5.    The reagent of any one of claims 1 to 4, wherein the concentration of said plexiform stabilizing means is optimised to preserve maximum analyte activity.

6.    The reagent of any one of claims 1 to 5, further comprising preservative means.

7.    The reagent of claim 6, wherein the preservative means in EDTA present in a final concentration of 0.1 to 1.5mM.

8.    The reagent of claim 6, wherein the preservative means is an antimicrobial agent.

9.    The reagent of claim 8, wherein the antimicrobial agent is chloramphericol.

10.    The reagent of any one of claims 1 to 9, wherein the biological analytes include protein and non-protein analytes, which is useful as a multiparameter control reagent.

11.    The reagent of claim 10, wherein the aqueous solution comprises substantially normal blood having all normally present proteins, enzymes and other analytes but with substantially all red blood cells, white blood cells and platelets removed.

0130708

12. The reagent of claim 10 or claim 11, further comprising additional proteins, enzymes or other analytes, whereby the reagent is useful as a multiparameter, abnormal control reagent.

13. The reagent of claim 10 or claim 11 having known amounts of proteins, enzymes and other analytes therein, whereby the reagent is useful as a multiparameter standard reagent.

14. The reagent of any one of claims 1 to 9, wherein the biological analyte is an isoenzyme which is present at a diagnostically significant concentration.

15. The reagent of claim 14, wherein the isoenzyme is isolated from tissue.

16. The reagent of claim 15, wherein the tissue is human tissue.

17. The reagent of any one of claims 14 to 16, further including other protein and non-protein analytes.

18. The reagent of any one of claims 14 to 17, further including human serum.

19. The reagent of any one of claims 14 to 18, further including a non-phosphate containing buffer.

20. The reagent of any one of claims 14 to 19, further including stabilizing cofactor means for maximising enzyme activity and chelating agent means, as necessary, to substantially bond with metallic ions, if present, for preserving enzyme binding site activity.

21. The reagent of any one of claims 14 to 20, wherein the isoenzyme is CK isoenzyme, LDH isoenzyme, ALT isoenzyme or AST isoenzyme.

22. The reagent of any one of claims 10 to 21, wherein the activity of the biological analyte in solution is maintained for at least ten days.

23. The reagent of any one of claims 10 to 22,

-66-

wherein the plexiform stabilizing means is present at a final volume concentration of from 2 to 8%

24. The reagent of claim 23, wherein the plexiform stabilizing means is maltose, mannitol, cellobiose or lactose.

25. The reagent of any one of claims 1 to 9, useful as a plasma control reagent, wherein the aqueous solution is plasma having therein anticoagulant means whereby clotting components are held intact and fibrin formation is inhibited.

26. The reagent of claim 25, wherein the plasma is human plasma.

27. The reagent of claim 25 or claim 26, wherein the anticoagulant means is citrate, oxalate, EDTA, heparin, or a combination thereof.

28. The reagent of any one of claims 25 to 27, wherein the plexiform stabilizing means is present at a final volume concentration of from 3 to 10%.

29. The reagent of any one of claims 25 to 28, useful as a coagulation control reagent, wherein said plasma may be manipulated to adjust specific clotting Factor or Factors in accordance with the type of coagulation control reagent desired.

30. The reagent of claim 29 wherein Factor I, Factor VIII, or a combination thereof are removed from the plasma.

31. The reagent of claim 29 or claim 30, wherein the plexiform stabilizing means is sucrose, lactose, mannitol, cellobiose or maltose.

32. The reagent of any one of claims 25 to 28, wherein the plasma has a known renin level, whereby the reagent is useful as a plasma renin control reagent useful for angiotensin testing.

33. The reagent of claim 32, wherein the plexiform

-67-

stabilizing means is glucose, maltose, mannitol, cellobiose or lactose.

34. The reagent of any one of claims 1 to 9, wherein the aqueous solution comprises a suspension of a processed tissue having a desired specific steroid receptor activity and contains a binding site protector for substantially preserving the receptor's activity and activating cofactor means, as necessary, for activating the receptor site.

35. The reagent of claim 34, wherein the tissue has estrogen and progesterone receptors.

36. The reagent of claim 35, wherein the binding site protector is sodium molybdate or sodium vanadate.

37. The reagent of claim 35 or claim 36, wherein the activating cofactor means is an active sulfhydryl compound.

38. The reagent of claim 37, wherein the sulfhydryl compound is dithiothreitol.

39. The reagent of any one of claims 34 to 35, wherein the processed tissue is tissue cytosol.

40. The reagent of any one of claims 34 to 39, wherein the solution contains a non-phosphate buffer.

41. The reagent of claim 40, wherein the buffer is HEPES buffer.

42. The reagent of claim 40 or claim 41, wherein the final concentration of the buffer is 25 to 150mM.

43. The reagent of any one of claims 34 to 42, further comprising an amount of inactive protein added, as necessary, to provide a predetermined total amount of protein per unit volume of reagent.

44. The reagent of claim 43, wherein the total amount of protein is between 2 and 4 mg/ml.

45. The reagent of claim 43 or 44, wherein the inactive protein is mature bovine uterine cytosol.

46. The reagent of any one of claims 34 to 45, wherein the plexiform stabilizing means is glucose, sucrose, lactose or a combination thereof.

47. The reagent of any one of claims 35 to 46, wherein the plexiform stabilizing means is present at a final volume concentration of from 50 to 400mM.

48. The reagent of any one of claims 1 to 47, wherein the plexiform stabilizing means is lactose.

49. The reagent of any one of claims 1 to 48, wherein the plexiform stabilizing means is present in a final volume concentration of 6%.

50. A reagent kit comprising a first vial containing a reagent according to claim 3 or claim 4 or any claim dependent on these claims, and a second vial containing a suitable quantity of liquid for reconstituting the reagent in the first vial.

51. A method of stabilizing an aqueous solution of biological analyte comprising adding thereto sufficient plexiform stabilizing means selected from glucose, sucrose, reducing monosaccharide sugars and reducing disaccharide sugars to obtain substantially increased analyte stability over that obtained without such plexiform stabilizing means.

52. The method of claim 51, wherein sufficient plexiform stabilizing means to give from 2 to 10 final volume percent thereof in the solution is added.

53. A method of producing the reagent of any one of claims 1 to 49, comprising adding to the aqueous solution of biological analyte sufficient plexiform stabilizing means selected from glucose, sucrose, reducing monosaccharide sugars and reducing disaccharide sugars to obtain substantially increased analyte stability over that obtained without such plexiform stabilizing means, and further comprising, where necessary or desired, the steps of:
removing substantially all the water from the reagent;
optimising the amount of plexiform stabilizing means added to preserve maximum analyte activity;
adding, as necessary or desired, preservative means, additional proteins, enzymes or other analytes, human serum, non-phosphate buffer, stabilizing cofactor means, chelating agent means, anticoagulant means, binding site protector, activating cofactor means or inactive protein; or
manipulating the solution to adjust specific clotting Factor or Factors.

54.    The method of any one of claims 51 to 53 further comprising the step of removing substantially all the water from the solution after addition of the plexiform stabilizing means.

55.    The method of claim 54, wherein the water is removed by lyophilization.

FIG-1

LACTOSE PILOTS
4°C LIQUID STABILITY OF
LACTATE DEHYDROGENASE

5% LACTOSE
8% LACTOSE
6% LACTOSE
4% LACTOSE
2% LACTOSE
CONTROL

LDH/UNITS PER LITER

DAYS

**FIG-2**

OPTICAL CLARITY AND STABILITY OF LACTOSE PILOTS AS MEASURED ON SERUM SAMPLES @4°C

■ AFTER 10 DAYS

□ AFTER 5 DAYS

○ AFTER 2 DAYS

● READING TAKEN IMMEDIATELY AFTER RECONSTITUTION

y-axis: ABSORBANCE @640nm (2.0, 1.8, 1.6, 1.4, 1.2, 1.0, .8, .6, .4, .2, 0)

x-axis: PERCENT LACTOSE (0, 2, 4, 6, 8)

FIG-3 | 4°C LIQUID STABILITY OF CREATININE LACTOSE PILOT STUDY

European Patent
Office

**EUROPEAN SEARCH REPORT**

Application number

EP 84303827.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | US - A - 4 127 502 (C.M. LIMUTTI et al.) <br><br> * Column 2, line 40 - column 3, line 7; column 4, lines 20-63 * | 1,2,3,4, 5,10,12, 13,51,52, 53,54,55 | G 01 N 33/96 <br> G 01 N 33/50 <br> C 12 Q 1/32 <br> C 12 Q 1/34 <br> C 12 Q 1/48 |
| D,X | US - A - 4 056 484 (N. HEINBURGER <br><br> * Column 2, lines 1-18; column 2, line 67 - column 3, line 38 * | 1,2,3,4, 5,25,26, 27,28,30, 31,51,52, 53,54,55 | |
| D,X | US - A - 3 527 331 (A. DEUTSCH) <br><br> * Column 1, lines 66-72; column 4, line 25 - column 5, line 29; abstract * | 1,3,14,19, 20,21,24, 50,51 | |
| X | GB - A - 2 049 929 (COULTER ELECTRONICS) <br><br> * Abstract * | 1,6,8 | **TECHNICAL FIELDS SEARCHED (Int. Cl. ³)** <br><br> G 01 N 33/00 |
| D,A | CANCER, vol. 46, no. 12, 1980, Philadelphia <br><br> BENRAAD et al. "Estradiol Receptor Activity in Lyophilized Calf Uterus and Human Breast Tumor Tissue" pages 2762-2764 <br><br> * Page 2762 * | 34,35, 39 | |

The present search report has been drawn up for all claims

| Place of search | Date of completion of the search | Examiner |
|---|---|---|
| VIENNA | 12-09-1984 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

| Category | DOCUMENTS CONSIDERED TO BE RELEVANT | | CLASSIFICATION OF THE APPLICATION (Int. Cl. ) |
|---|---|---|---|
| | Citation of document with indication, where appropriate, of relevant passages | Relevant to claim | |
| D,A | CANCER, vol. 46, no. 12, 1980, Philadelphia<br><br>BOJAR et al. "Investigation of Steroid Hormone Receptors in Lyophilized Calf Uterine Tissue Powder"<br>pages 2770-2774<br><br>  * Page 2771 *<br>-- | 34,35, 39 | |
| D,A | JOURNAL OF CLINICAL CHEMISTRY AND CLINICAL BIOCHEMISTRY, vol. 19, no. 8, 1981, Berlin, New York<br><br>VERMOUSEK et al. "Stable Standard for Determination of Oestrogen Receptor"<br>page 865<br><br>  * Page 865 *<br>---- | 36,37, 38 | TECHNICAL FIELDS SEARCHED (Int. Cl.³) |

EPO Form 1503.2  06.78